# EUROPEAN PATENT APPLICATION

(11) **EP 4 789 703 A1**
(43) Date of publication of application: **12.08.2026**
(21) Application number: 26156776.2
(22) Date of filing: 06.02.2026
(51) Int. Cl.: A61M 5/34

(54) **LED CURING WITH UV-C**

(30) Priority: 06.02.2025 US 202563754854 P
(71) Applicant: ATS Corporation, Cambridge, Ontario N3H 4R7 (CA)
(72) Inventor: KOHLMEIER, Christoph, 85551 Heimstetten (DE); LINDNER, Roland, 85551 Heimstetten (DE)
(74) Representative: Bardehle Pagenberg Partnerschaft mbB Patentanwälte Rechtsanwälte

(57) **Abstract**

A method for curing adhesive during assembly of a glass syringe, the method comprising the following steps: a.) providing an adhesive, a glass barrel and a needle;
b.) dispensing the adhesive onto the glass barrel and/or needle;
c.) arranging the needle onto the barrel; and thereafter:
curing the adhesive by means of a UVC LED radiation source.

## Description

### 1. Field of the invention

This invention relates to a method for curing adhesive during assembly of a glass syringe.

### 2. Background

The invention relates to methods for assembling glass syringes, specifically focusing on curing adhesives that bond the needle to the glass barrel. This process is important for producing syringes with high precision, durability, and reliability, as they are often used in medical applications requiring sterility and mechanical strength. Traditional methods of curing adhesives often rely solely on UV light or thermal curing, which can be slow, inconsistent, or unsuitable for adhesives requiring specific curing conditions. Furthermore, traditional approaches may not ensure uniform bonding, leading to defects or weak adhesion, which compromise syringe performance. Another common error in syringe production is an undesired at least partially sticking surface of the adhesive.

The primary problem addressed by this invention is the need for a faster, more reliable, and efficient adhesive curing process for syringe assembly that ensures consistent quality and strong adhesion between the needle and barrel. Conventional curing techniques may result in incomplete curing due to inadequate radiation or heating, uneven application of energy, or temperature-sensitive materials. These limitations increase production time and costs, reduce product reliability, and raise the likelihood of syringe failure during use.

In view of the foregoing, there is a need for an improved curing method for syringes. It is thus an object of the present invention to overcome some or all the deficiencies of the prior art. The invention solves this problem by combining UVC LED radiation and IR heating, with options for additional UVA and UVB radiation. This dual-action approach ensures thorough curing of the adhesive by targeting both surface and internal layers while optimizing thermal properties. Features such as preheating, temperature control, and precise timing further enhance the process, enabling faster, more uniform, and efficient syringe assembly. By addressing the shortcomings of traditional methods, the invention provides a robust solution for high-quality syringe production with improved reliability and efficiency.

### 3. Summary

The above objects are at least partially achieved by the subject matter of independent claim 1. Preferred embodiments are the subject of the dependent claims, and the skilled person will find clues to other suitable aspects of the present invention in the overall disclosure of the present application.

An aspect of the invention relates to a method for curing adhesive during assembly of a glass syringe, the method comprising the following steps: a.) providing an adhesive, a glass barrel and a needle; b.) dispensing the adhesive onto the glass barrel and/or needle; c.) arranging the needle onto the barrel; and thereafter: curing the adhesive by means of a UVC LED radiation source. The method may further comprise heating the adhesive, for example by means of an IR radiation source and/or a hot-air heating source, wherein the heating is preferably at least partially carried out simultaneously with the UVC LED radiation.

With such a method a glass syringe barrel is prepared with a medical-grade adhesive applied to its surface where it will connect to a steel needle. After positioning the needle, the adhesive is exposed to a UVC LED light source to initiate rapid curing. Simultaneously and/or shortly after and/or before, IR radiation heats the adhesive to enhance the curing process, ensuring strong adhesion between the needle and the barrel.

It will be understood that the sequence of steps b.) and c.) is not limited to the order as recited. In alternative embodiments, the needle may first be arranged onto the barrel, and the adhesive may be dispensed thereafter.

The method according to the invention is suitable for use with a variety of UV-curable adhesives. In one example, the adhesive may be Loctite^{™} 3345, which is an adhesive that has received market approval as a packaging material for pharmaceutical applications and is widely used in glass syringe assembly. However, the invention is not limited to any particular adhesive formulation, and the skilled person will appreciate that the method may be applied to any other UV-curable adhesives.

In another embodiment, before dispensing the adhesive in step b), the glass barrel is preheated using hot air and/or an infrared (IR) radiation source to bring its surface to an optimal temperature. This preheating step can for instance ensure that the adhesive, when applied, spreads evenly and adheres more effectively to the glass surface. By preheating the barrel, one advantage is that the adhesive's viscosity is reduced upon contact, allowing it to flow more uniformly and penetrate micro-irregularities on the glass surface. This step can - among other advantages - enhance the bonding strength, improve curing efficiency, and ensure greater consistency in the later steps of the syringe assembly process.

The combination of UVC and IR techniques ensures a faster and more reliable bond compared to traditional curing methods like UV light alone. This dual-action process minimizes assembly time, improves production efficiency, and results in a durable bond capable of withstanding the mechanical and thermal stresses typical of syringe usage.

Further improvement can be achieved when in addition to the UVC LED radiation source, an UVA LED radiation source, and/or an UVB LED radiation source is applied for curing the adhesive.

With such a method, after the adhesive is applied and the needle is positioned on the glass syringe barrel, the curing process is initiated using a combination of UVC, UVA, and/or UVB LED radiation sources. For instance, the UVC radiation could provide a quick and superficial cure, while UVA and UVB radiation could contribute to deep curing of the adhesive. Generally, UVA radiation reaches deeper layers than UVB radiation. Such a multi-spectrum approach ensures a more thorough and uniform curing process, reducing the likelihood of uncured areas that could weaken the bond. By applying multiple UV wavelengths, manufacturers can adapt the curing process to different adhesive formulations, resulting in greater flexibility, improved product quality, and optimized curing efficiency.

In another embodiment, after the adhesive is applied and the needle is positioned on the glass syringe barrel, the curing process is carried out using a combination of IR heating, UVA LED, and UVC LED radiation sources. The UVC LED radiation enhances surface curing of the adhesive, while the UVA LED radiation enables deep curing of the adhesive. Meanwhile, IR radiation is used to maintain an optimal adhesive surface temperature during the curing process, preventing thermal stress while enhancing the curing reaction. This combination of thermal activation and multi-wavelength UV curing ensures fast, complete, and reliable bonding, optimizing both adhesive performance and production efficiency.

Further improvement can be achieved when before curing the adhesive, the adhesive is heated in a preheating step.

With such a method after applying the adhesive to the glass syringe barrel and/or needle and before curing begins, the adhesive is preheated using an infrared (IR) heating source. This preheating step can soften the adhesive, reduces its viscosity, and allows it to spread more evenly across the bonding surfaces. The preheating step ensures better adhesion and penetration, which can lead to a stronger and more reliable bond. Additionally, preheating can accelerate the subsequent curing process, reducing total assembly time and improving production throughput. Such a method is advantageous for adhesives with high viscosity or those requiring precise flow characteristics before curing.

This method can be further improved when the adhesive is heated for 2 to 8 seconds, preferably 2 to 6 seconds, most preferably for 2 to 4 seconds.

Such a controlled heating duration can ensure the adhesive reaches the ideal temperature for spreading and priming without degrading its chemical properties or causing premature curing. By optimizing the preheating time, the method achieves consistent results across production batches. Heating for this duration ensures the adhesive remains manageable and enhances its bonding performance during the subsequent curing process. This approach also reduces variability in assembly and minimizes the risk of errors, leading to higher-quality syringes and improved manufacturing efficiency.

This method can be further improved when the temperature during heating is varied, preferably in a temperature range between 110°C and 180°C.

With such a method, the adhesive applied to the syringe barrel and/or needle is preheated using IR radiation, where the temperature is gradually increased during the 4-second heating duration. For example, the temperature might start at 110°C and gradually rise to 180°C, ensuring the adhesive transitions smoothly from a viscous state to a more pliable and evenly distributed layer. Varying the temperature during heating can optimize the adhesive's properties, such as its flow behavior and interaction with the syringe materials. This method enhances the adhesive's ability to fill gaps and adhere to irregular surfaces, resulting in a stronger, more uniform bond. Additionally, a variable temperature profile can help prevent thermal stress on the syringe components, ensuring consistent quality and durability in the final product.

This method can be further improved when the temperature variation is done with a gradient ΔT/Δt in the range of from 5 K/min to 50 K/min, preferably from 10 K/min to 40 K/min, more preferably from 20 K/min to 30 K/min.

With such a method the adhesive applied to the syringe barrel and/or needle is preheated using a controlled temperature gradient. For example, the temperature may increase at a rate of 25 K/min, starting at 100°C and reaching 105°C within a heating duration of 12 seconds. This gradual and precise increase can ensure the adhesive softens uniformly, avoiding rapid thermal changes that could compromise its properties. By controlling the temperature gradient within the specified range, the method ensures optimal heating dynamics, allowing the adhesive to achieve ideal flow characteristics without degradation or premature curing. This approach is beneficial for adhesives with temperature-sensitive chemistries or when working with delicate materials.

Further improvement is achieved when the radiation is provided in an angle of incidence in the range 0° to 60°, preferably in the range 15° to 45°, most preferred about 35°.

With such an angle of incidence during the curing process, UVC LED radiation (and optionally UVA or UVB) is directed at the adhesive on the syringe barrel and/or needle at an angle of for instance 35°. This angle ensures that the radiation penetrates effectively, reaching all parts of the adhesive, including areas that are difficult to access. Applying radiation at such an optimized angle can improve the uniformity of the curing process, ensuring full activation of the adhesive across its entire surface and depth. This approach can be useful in complex geometries, such as the joint between the needle and barrel, where straight-on radiation without an incidence angle might leave shadowed or under-cured regions. By enhancing the curing consistency, the method ensures stronger bonds, reduced defects, and higher overall quality of the assembled syringes. The angle of incidence is defined by the angular deviation of an axis perpendicular to the needle axis at the adhesive

Further improvement can be achieved when the radiation is provided by a radiation source, and the shortest distance between the radiation source and the adhesive is in the range between 5 mm and 40 mm, preferably in the range 7 mm to 30 mm, most preferably around 10 mm.

With such a method a UVC LED radiation source is positioned for instance 10 mm from the adhesive applied to the syringe barrel and/or needle. This precise positioning ensures optimal energy delivery, allowing the radiation to cure the adhesive effectively without overexposure or underexposure. Maintaining the specified distance is important for achieving consistent and efficient curing. If the radiation source is too far, the intensity may decrease, leading to incomplete curing. If it is too close, the adhesive or syringe materials may overheat or degrade. By keeping to the specified range, the method ensures uniform curing, strong adhesive bonds, and high-quality syringe assembly.

Further improvement is achieved when the wavelength of the UVC radiation is in the range 100 nm to 280 nm, preferably in the range 220 nm to 280 nm, most preferably in the range 240 nm to 280 nm.

With such a method the adhesive on the syringe barrel and/or needle is cured using UVC radiation with a wavelength of for instance 280 nm, which is effective for initiating photochemical reactions in common adhesive formulations. This wavelength is selected to maximize energy absorption by the adhesive, ensuring rapid and thorough curing.

Using UVC radiation within this specified wavelength range is advantageous because it targets the adhesive's photoinitiators precisely, leading to efficient cross-linking and stronger bonds. Additionally, the chosen wavelength minimizes the risk of thermal damage to the syringe components while optimizing the curing process. This results in faster production cycles, consistent product quality, and reliable performance of the assembled syringes.

Further improvement is achieved when the intensity of the UVC radiation is in the range 100 mW/cm² to 3000 mW/cm², preferably in the range 300 mW/cm² to 1500 mW/cm².

With such a method the adhesive applied to the syringe barrel and/or needle is cured using UVC radiation at an intensity of for instance 300 mW/cm². This intensity can provide sufficient energy to activate the adhesive's photoinitiators effectively without causing damage to the adhesive or the syringe components. Maintaining the specified intensity range ensures a balanced curing process-too low of an intensity may lead to incomplete curing, while too high of an intensity could overheat the adhesive or surrounding materials. By operating within this range, the method ensures optimal adhesive performance, producing strong, durable bonds. Additionally, the specified intensity contributes to faster production times and improved consistency in syringe assembly quality. An increase of the UVC radiation to higher values, for example to 2000 mW/cm² or even 3000 mW/cm² could make the method even more time efficient by decreasing the curing time.

Further improvement is achieved when the radiation is provided for a duration between 3 and 40 seconds, preferably between 8 and 30 seconds, more preferably between 10 and 25 seconds, most preferably around 20 seconds.

With such a method the adhesive applied to the syringe barrel and/or needle is cured using UVC radiation for instance for 20 seconds. Such a duration is carefully calibrated to ensure the adhesive fully cures, achieving maximum bond strength without overexposure that might degrade the adhesive or syringe materials. By controlling the exposure time within this range, the method balances curing efficiency and material safety. Shorter durations might lead to incomplete curing, weakening the bond, while longer durations could waste energy or cause thermal stress. The specified time ensures consistent results, optimized production cycles, and high-quality syringe assemblies capable of withstanding mechanical and thermal stress during use.

Further improvement is achieved when the heating is carried out such that the adhesive during the heating has a surface temperature in the range of between 100 °C to 250°C, preferably between 110 °C and 200°C, more preferably between 110 °C and 180°C.

With such a method the adhesive applied to the syringe barrel and/or needle is heated to a surface temperature in the range between 110°C and 180 °C during the preheating and/or simultaneous heating process. This temperature allows the adhesive to achieve an optimized viscosity and reactivity, ensuring uniform application and efficient curing when exposed to UVC radiation. By maintaining the adhesive's temperature within the specified range, the method can prevent thermal degradation of the adhesive while ensuring optimal flow and bonding characteristics. This controlled heating improves the adhesive's penetration into surface irregularities, resulting in a strong and durable bond.

Further improvement is achieved when the curing of the adhesive is by two UVC radiation sources and at least one heating source, such as an IR radiation source or a hot-air heating source.

With such a method two UVC LED sources are positioned at different angles to evenly expose the adhesive on the syringe barrel and/or needle, ensuring comprehensive curing. Simultaneously, an IR radiation source provides controlled heat to enhance the adhesive's curing reaction. For example, the two UVC sources might operate at a wavelength in the range of 275 nm to 278 nm, preferably at 278 nm while the IR source maintains a surface temperature in the range of around 110°C to 180°C. Using multiple UVC sources can ensure complete and uniform curing, reducing the likelihood of shadowed or under-cured areas. The addition of an IR radiation source further accelerates the process by optimizing the adhesive's thermal reactivity. This combination achieves faster curing times, stronger adhesive bonds, and higher production efficiency, all while ensuring consistent quality in syringe assembly.

The curing process can also utilize two or more UVC LED radiation sources, preferably ranging from two to four, in combination with one IR radiation source. The UVC radiation sources can be positioned strategically to provide comprehensive and uniform curing, ensuring that the adhesive on the syringe barrel and/or needle receives optimal radiation exposure: To accommodate for instance high-throughput manufacturing, the UVC sources can be arranged in a way that does not obstruct the entry and exit path of syringes transported by movers on a conveyor system. Further improvement is achieved when the surface of the adhesive is dry after the curing.

With such a method after applying the adhesive to the syringe barrel and/or needle and curing it with UVC and IR radiation, the surface of the adhesive is dry and non-sticky. Such a dry state can ensure that the adhesive has undergone complete photochemical and thermal reactions, forming a durable bond. Achieving a dry surface is important for ensuring the integrity of the syringe assembly, as it prevents smearing, contamination, or unintended adhesion to other components during handling. Additionally, a dry adhesive surface can enhance the syringe's usability and ensures it meets strict quality and safety standards for medical applications. This method contributes to reliable performance and durability in the final product.

Further improvement is achieved when after step b.) and before curing the adhesive, the adhesive is pre-cured.

With such a method after the adhesive is applied to the syringe barrel and/or needle, it undergoes a pre-curing phase using for instance low-intensity UVC (or optionally UVA and/or UVB) radiation or mild heating. For instance, the adhesive might be exposed to UVC radiation at a lower intensity for a brief period, enough to partially solidify its surface without fully curing it. Pre-curing stabilizes the adhesive, ensuring it stays in place when the needle is positioned or during handling before the final curing. This step prevents the adhesive from dripping or spreading unevenly, leading to more precise and controlled assembly. Additionally, pre-curing enhances the effectiveness of the final curing step by ensuring the adhesive maintains optimal contact with the syringe components, resulting in stronger bonds.

In another embodiment, the temporary fixation of the needle is achieved by a pre-curing step, for instance as taught in US 12,110,253 B2. After dispensing the adhesive, it is partially cured using a UV LED light source, such as a UV LED spot. The light can be directed longitudinally along the glass barrel toward the adhesive area. This pre-curing step can initiate a bond and can bring the adhesive to a gel-like consistency, which can be sufficient to hold the needle in its position without an external gripper. Subsequently, the gripper can be removed before the final curing step, which for instance prevents the gripper from casting a shadow on the adhesive and ensures a more uniform final cure.

In another embodiment, the needle can be temporarily fixed to the barrel by mechanical means, thus avoiding the need for a pre-curing step for fixation. For example, as described in the patent US 4,795,445 A, the glass barrel can be formed with a spout that terminates in a circumscribing lip. This lip is designed to form a controlled friction fit with the needle hub, thereby holding the needle axially in place. The needle is then permanently secured by the subsequent final curing of the adhesive.

### 4. Brief description of the figures

In the following, preferred embodiments of the disclosure are disclosed by reference to the accompanying figures.
- Fig. 1:: illustrates the method according to the invention in a schematic view.
- Fig. 2:: shows experimental data of the adhesive surface temperature during the heating in a t-T-diagram.
- Fig. 3:: shows an exemplary production setup to implement the method according to the invention.

### 5. Detailed description of the figures

The subsequent sections provide a detailed description of the invention, referencing the accompanying illustrations for clarity. The descriptions represent examples only and are not intended to limit the invention's scope. Identical reference numerals across the figures and text denote the same components. The illustrations may not reflect actual size or scale; their dimensions, proportions, and depictions of elements might be enhanced for better understanding and visual convenience.

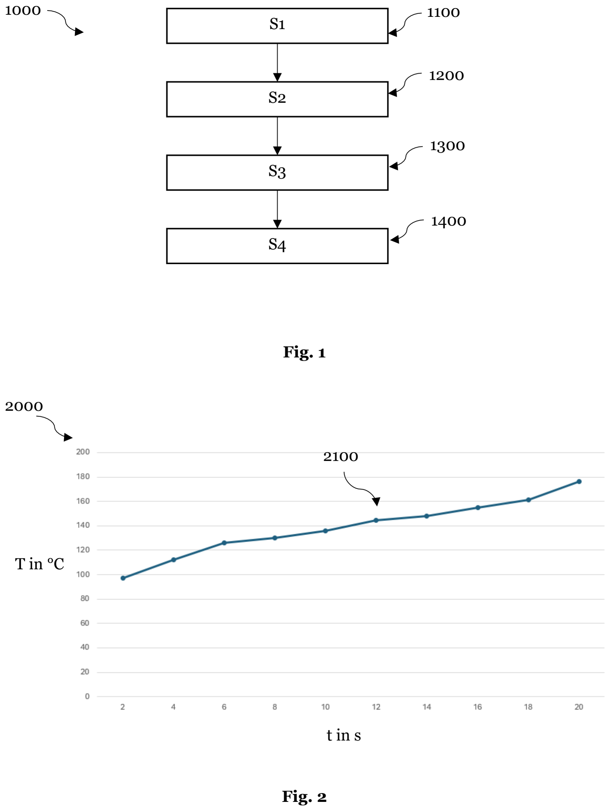

Figure 1 illustrates a method 1000 for curing adhesive during assembly of a glass syringe according to the invention in a schematic view. In a first step 1100 an adhesive, a glass barrel and a needle are provided. A second step 1200 follows, where the adhesive is dispensed onto the glass barrel and/or needle. In a third step 1300, the needle is arranged onto the barrel. Afterwards follows a fourth step 1400, where the adhesive is cured by means of a UVC LED radiation source and the adhesive is heated by means of IR radiation, and wherein the heating is preferably at least partially simultaneously carried out with the UVC LED radiation.

Figure 2 shows experimental data of the adhesive surface temperature during the heating in a t-T-diagram 2000. The adhesive temperature profile observed when using an Infra-Red (IR) heating system is illustrated herein. The temperature measurements 2100 were obtained using an IR camera, calibrated with an approximate emissivity coefficient corresponding to the adhesive. The heating is carried out such that the adhesive during the heating has a surface temperature in the range of between 97 °C to 177°C.

The temperature remains within a controlled range of 97°C to 177°C, ensuring optimal conditions for the adhesive's viscosity and curing properties. The heating profile ensures that the adhesive is brought to an ideal temperature range for proper flow, penetration, and curing without overheating or degrading. The use of IR heating, combined with careful temperature control, minimizes thermal stress on the adhesive and syringe components.

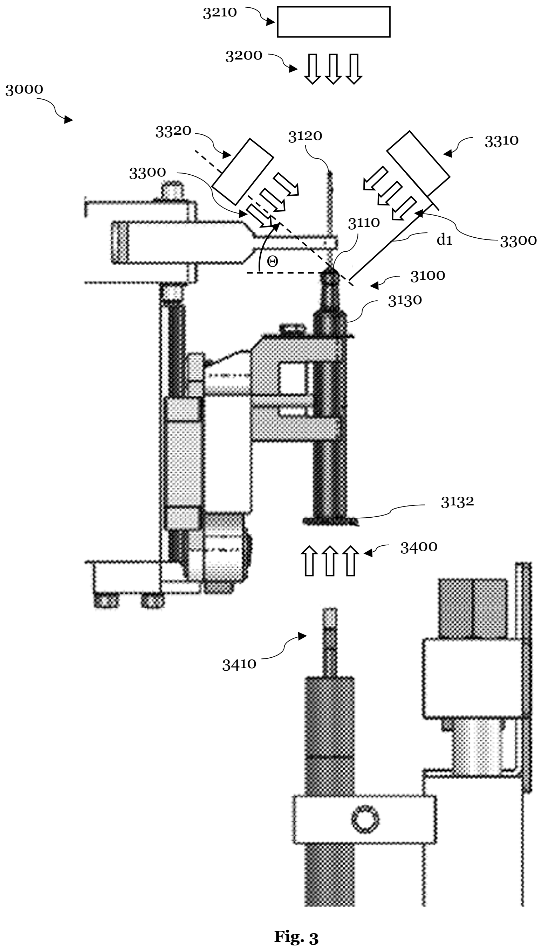

Figure 3 shows an exemplary production setup 3000 to implement the method according to the invention. In the production setup 3000, the syringe 3100 is assembled by arranging the needle 3120 onto the glass barrel 3130 (i.e., inserting one end of the needle into the barrel) and dispensing adhesive 3110 onto the needle 3120 and the glass barrel 3130. An optional IR radiation source 3210 is arranged directly opposite the needle 3120, such that IR radiation 3200 is emitted onto the adhesive.

Additionally, a first UVC radiation source 3310 and a second UVC radiation source 3320 are arranged on opposite sides of the syringe 3100, such that UVC radiation 3300 is emitted onto the adhesive at an angle of incidence Θ of about 35° from two sides. As can be seen in Figure 3, the angle of incidence is defined by the angular deviation of an axis orthogonal to the needle axis at the adhesive. In addition to the first and second UVC radiation sources 3310 and 3320, an UVA radiation source 3410 is applied for curing the adhesive. The UVA radiation source is arranged on the side of the finger flange 3132 of the syringe 3100, emitting the UVA radiation 3400 into the glass barrel 3130. The glass barrel 3130 thus serves as a light guide to guide the UVA radiation to the adhesive 3110. This way, the UVA radiation gets refracted on the upper end of the glass barrel and thus exposes the adhesive from multiple sides.

The technical necessity of accompanying UVC radiation with UVA radiation arises from the material properties of the adhesive used. The adhesive applied for glass barrel syringes exhibits a shrinkage of approximately 10 % during the transition from the liquid to the solid state. UVC radiation penetrates only a few hundredths of a millimeter into the surface of the adhesive and cannot reach the underlying volumes. Exclusive exposure of the adhesive surface therefore results in a layer-like polymerization, accompanied by surface-level shrinkage, which creates a volumetric discrepancy between the upper layer and the deeper regions. This volumetric difference leads to crack-like surface structures and consequently to optical impairment.

By directing UVA radiation-specifically via the finger flange and through light refraction at the syringe tip-the lower adhesive volume is likewise stimulated to polymerize. As a result, the shrinkage-induced volumetric differences are compensated. Since both the surface and the underlying volumes polymerize simultaneously, internal stress differences are eliminated or significantly reduced, thereby preventing optical defects.

The shortest distance d1 between the UVC radiation sources and the adhesive is around 10 mm (not to scale in Fig. 3). In this embodiment the curing of the adhesive is by two UVC radiation sources, one UVA radiation source and one IR radiation source.

This production setup 3000 exemplifies an approach to syringe assembly according to the invention. The combination of UVC and UVA radiation ensures comprehensive curing of both surface and internal adhesive layers, while precise IR heating optimizes adhesive flow and reactivity, resulting in strong and uniform bonds. Furthermore, the application of multi-directional UVA radiation eliminates shadowed areas, minimizing the risk of incomplete curing and weak bonds, thereby ensuring minimal defects in the final product.

In the following, further preferred embodiments are described to facilitate understanding:
Embodiment 1. A method (1000) for curing adhesive (3110) during assembly of a glass syringe (3100), the method (1000) comprising the following steps:
   a.) providing an adhesive (3110), a glass barrel (3130) and a needle (3120);
   b.) dispensing the adhesive (3110) onto the glass barrel (3130) and/or needle (3120);
   c.) arranging the needle (3120) onto the barrel; and thereafter:
   curing the adhesive (3110) by means of a UVC LED radiation source (3310, 3320) and heating the adhesive (3110) by means of IR radiation (3200), wherein the heating is preferably at least partially simultaneously carried out with the UVC LED radiation (3300).
Embodiment 2. An apparatus for curing an adhesive during assembly of a glass syringe, the apparatus comprising: a support for holding the syringe, the syringe comprising a glass barrel and a needle joined by the adhesive; and at least one UVC LED radiation source arranged to emit UVC radiation onto the adhesive.
Embodiment 3. The apparatus of embodiment 2, further comprising at least one heating source, wherein the heating source is an IR radiation source and/or a hot-air heating source, and a controller configured to operate the heating source at least partially simultaneously with the UVC LED radiation source.
Embodiment 4. The apparatus of any preceding embodiment, further comprising at least one UVA LED radiation source and/or at least one UVB LED radiation source arranged to cure the adhesive.
Embodiment 5. The apparatus of any preceding embodiment, further comprising a heating source and a controller configured to operate the heating source to preheat the adhesive before final curing.
Embodiment 6. The apparatus of embodiment 5, wherein the controller is configured to operate the heating source for a duration of 2 to 8 seconds, preferably 2 to 6 seconds, most preferably for 2 to 4 seconds.
Embodiment 7. The apparatus of embodiment 5 or 6, wherein the heating source and the controller are configured to vary the temperature of the adhesive during heating, preferably within a temperature range between 110°C and 180°C.
Embodiment 8. The apparatus of any preceding embodiment, wherein at least one radiation source is arranged to provide radiation at an angle of incidence in the range of 0° to 60°, preferably in the range of 15° to 45°, most preferably about 35°.
Embodiment 9. The apparatus of any preceding embodiment, wherein at least one radiation source is positioned such that the shortest distance between the radiation source and the adhesive is in the range between 5 mm and 40 mm, preferably in the range of 7 mm to 30 mm, most preferably around 10 mm.
Embodiment 10. The apparatus of any preceding embodiment, wherein the at least one UVC LED radiation source is configured to emit radiation with a wavelength in the range of 100 nm to 280 nm, preferably in the range of 220 nm to 280 nm, most preferably in the range of 275 nm to 278 nm, most preferred 278 nm.
Embodiment 11. The apparatus of any preceding embodiment, wherein the at least one UVC LED radiation source is configured to provide an intensity in the range of 100 mW/cm² to 3000 mW/cm², preferably in the range of 300 mW/cm² to 1500 mW/cm².
Embodiment 12. The apparatus of any preceding embodiment, further comprising a controller configured to operate at least one radiation source for a duration between 3 and 40 seconds, preferably between 8 and 30 seconds, more preferably between 10 and 25 seconds, most preferably around 20 seconds.
Embodiment 13. The apparatus of any preceding embodiment comprising a heating source, wherein the heating source and a controller are configured to maintain a surface temperature of the adhesive during heating in the range of 100°C to 250°C, preferably between 110°C and 200°C, more preferably between 110°C and 180°C.
Embodiment 14. The apparatus of any preceding embodiment, comprising two UVC radiation sources and at least one heating source, wherein the heating source is an IR radiation source and/or a hot-air heating source.
Embodiment 15. The apparatus of any preceding embodiment, wherein the radiation sources, any heating sources, and a controller are configured to operate in such a way that the surface of the adhesive is dry after curing.
Embodiment 16. The apparatus of any preceding embodiment, wherein at least one radiation source and a controller are configured to operate the source in a pre-curing mode to pre-cure the adhesive before a final curing cycle.

### Reference list:

1000: method
1100: step 1
1200: step 2
1300: step 3
1400: step 4
2000: diagram
2100: temperature curve
3000: production setup
3100: syringe
3110: adhesive
3120: needle
3130: glass barrel
3132: finger flange
3200: IR radiation
3210: IR radiation source
3300: UVC radiation
3310: first UVC radiation source
3320: second UVC radiation source
3400: UVA radiation
3410: UVA radiation source
Θ: Angle of incidence

## Claims

1. A method (1000) for curing adhesive (3110) during assembly of a glass syringe (3100), the method (1000) comprising the following steps:
a.) providing an adhesive (3110), a glass barrel (3130) and a needle (3120);
b.) dispensing the adhesive (3110) onto the glass barrel (3130) and/or needle (3120);
c.) arranging the needle (3120) onto the barrel; and thereafter:
curing the adhesive (3110) by means of a UVC LED radiation source (3310, 3320).

2. The method (1000) according to claim 1, further comprising heating the adhesive (3110) by means of at least one heating source, wherein the heating source is an IR radiation source (3210) and/or a hot-air heating source and wherein the heating is preferably at least partially simultaneously carried out with the UVC LED radiation (3300).

3. The method (1000) according to claim 1 or 2, wherein in addition to the UVC LED radiation source (3310,3320), an UVA LED radiation source (3410), and/or an UVB LED radiation source is applied for curing the adhesive (3110).

4. The method (1000) according to any of the preceding claims, wherein before curing the adhesive (3110), the adhesive (3110) is heated in a preheating step.

5. The method (1000) according to claim 4, wherein the adhesive (3110) is heated for 2 to 8 seconds, preferably 2 to 6 seconds, most preferably for 2 to 4 seconds.

6. The method (1000) according to any one of the preceding claims 4 or 5, wherein the temperature during heating is varied, preferably in a temperature range between 110°C and 180°C.

7. The method (1000) according to any of the preceding claims, wherein the radiation is provided in an angle of incidence (Θ) in the range 0° to 60°, preferably in the range 15° to 45°, most preferred about 35°.

8. The method (1000) according to any of the preceding claims, wherein the radiation is provided by a radiation source (3210, 3310, 3320, 3410), and the shortest distance between the radiation source and the adhesive (3110) is in the range between 5 mm and 40 mm, preferably in the range 7 mm to 30 mm, most preferably around 10 mm.

9. The method (1000) according to any of the preceding claims, wherein the wavelength of the UVC radiation (3300) is in the range 100 nm to 280 nm, preferably in the range 220 nm to 280 nm, most preferably in the range 275 nm to 278 nm, most preferred 278 nm.

10. The method (1000) according to any of the preceding claims, wherein the intensity of the UVC radiation (3300) is in the range 100 mW/cm² to 3000 mW/cm², preferably in the range 300 mW/cm² to 1500 mW/cm².

11. The method (1000) according to any one of the preceding claims, wherein the radiation is provided for a duration between 3 and 40 seconds, preferably between 8 and 30 seconds, more preferably between 10 and 25 seconds, most preferably around 20 seconds.

12. The method (1000) according to any one of the preceding claims, wherein the heating is carried out such that the adhesive (3110) during the heating has a surface temperature in the range of between 100 °C to 250°C, preferably between 110 °C and 200°C, more preferably between 110 °C and 180 °C.

13. The method (1000) according to any one of the preceding claims, wherein the curing of the adhesive (3110) is by two UVC radiation sources and at least one heating source, wherein the heating source is an IR radiation source (3210) and/or a hot-air heating source.

14. The method (1000) according to any one of the preceding claims, wherein the surface of the adhesive (3110) is dry after the curing.

15. The method (1000) according to any one of the preceding claims, wherein after step b.) and before curing the adhesive (3110), the adhesive (3110) is pre-cured.
